(19) Europäisches Patentamt

European Patent Office

Office européen des brevets

(11) **EP 1 640 708 A1**

(12) **EUROPÄISCHE PATENTANMELDUNG**

(43) Veröffentlichungstag:
    **29.03.2006 Patentblatt 2006/13**

(51) Int Cl.:
    *G01N 21/35* (2006.01)    *G01N 21/37* (2006.01)

(21) Anmeldenummer: **04022907.2**

(22) Anmeldetag: **25.09.2004**

(84) Benannte Vertragsstaaten:
    **AT BE BG CH CY CZ DE DK EE ES FI FR GB GR
    HU IE IT LI LU MC NL PL PT RO SE SI SK TR**
    Benannte Erstreckungsstaaten:
    **AL HR LT LV MK**

(71) Anmelder: **MAIHAK AG**
    **22399 Hamburg (DE)**

(72) Erfinder:
    • **Olsowski, Wolfgang, Dr.**
      **21407 Deutsch Evern (DE)**

    • **Zöchbauer, Michael, Dr.**
      **61440 Oberursel (DE)**

(74) Vertreter: **Ludewigt, Christoph
    Sick AG,
    Intellectual Property,
    Sebastian-Kneipp-Strasse 1
    79183 Waldkirch (DE)**

(54) **Zweistrahl-Gasanalysator**

(57)    Die Erfindung betrifft einen Zweistrahl-Gasanalysator mit einer Messstrahlungsquelle, mit einer ein zu messendes Gas aufnehmbaren Messküvette, mit einer ein Referenzgas enthaltenden Referenzküvette, mit einem Detektor zur Detektion der von der Messstrahlungsquelle abgegebenen Messstrahlung, mit einem Modulator zum periodischen Umschalten der Messstrahlung von der Messseite auf die Referenzseite und umgekehrt und mit einer Auswerteeinheit. Um einen verbesserten Zwei-strahl-Gasanalysator bereitzustellen, der eine hohe Langzeitstabilität von Nullpunkt und Empfindlichkeit aufweist, wird vorgeschlagen, dass der Modulator derart ausgebildet ist, um innerhalb einer Periode wenigstens zwei Dunkelphasen zu erzeugen. Die Erfindung betrifft auch ein entsprechendes verbessertes Verfahren zur Bestimmung von Gaskomponenten in einem Messgas mit dem Zweistrahl-Verfahren, bei dem wenigstens zwei Dunkelphasen vorgesehen sind, während denen die Messstrahlung blockiert wird.

Fig. 1

EP 1 640 708 A1

**Beschreibung**

Die Erfindung betrifft einen Zweistrahl-Gasanalysator gemäß dem Oberbegriff des Anspruchs 1.

Aus der DE 195 47 787 C1 ist ein gattungsgemäßer Zweistrahl-Gasanalysator bekannt, der nach dem NDIR-Verfahren (non-dispersiveinfrared) arbeitet. Dabei handelt es sich um ein Fotometer mit einer Infrarot-Strahlungsquelle, deren Messstrahlung mittels eines Blendenrades entweder einer Messküvette, die das zu messende Gas enthält, oder einer Referenzküvette, die ein Referenzgas enthält, zugeführt wird. Das Blendenrad schaltet periodisch zwischen der Messseite und der Referenzseite um. Die Infrarotstrahlung durchdringt die jeweilige Küvette und wird von einem Strahlungsdetektor detektiert. Bei dem Detektor handelt es sich um einen opto-pneumatischen Detektor mit zwei gleich dimensionierten Kammern, die mit dem Gas abgefüllt sind, das der zu messenden Komponente entspricht. Zwischen den beiden Detektorkammern ist ein Messfühler angebracht, der auf die Druckdifferenz durch die in den beiden Kammern erzeugten Druck- und Temperaturänderungen anspricht. Wenn kein Messgas in der Messküvette vorhanden ist, ist die Strahlungsabsorption und damit die erzeugte Druck- und Temperaturänderung in beiden Empfängerkammern gleich. Differenzen entstehen, wenn in der Messküvette ein Teil der Strahlung vorabsorbiert wird.

Zwar hat dieser bekannte Zweistrahl-Gasanalysator den Vorteil, dass zur Nachkalibrierung die Messküvette nicht mit einem Kalibrier- oder Eichgas gefüllt werden muss, dennoch ist aufgrund von Intensitätsverlusten durch Verschmutzung ein permanentes Nachkalibrieren notwendig. Dazu stellt die DE 195 47 787 C1 ein relativ aufwendiges Nachkalibrierverfahren zur Verfügung.

Aus der DE 31 37 658 C2 ist ein Einstrahl-Gasanalysator bekannt, bei dem zur Verbesserung der Messgenauigkeit zwei verschiedene Filter über ein Blendenrad in den Strahlengang geschwenkt werden, so dass durch eine geschickte Auswertung der modulierten Messergebnisse eine bessere Genauigkeit erreicht werden kann.

Derartige Gasanalysatoren werden verwendet in der Emissionsmessung, Rauchgasmessung, Abgasmessung oder in chemischen Prozessen. Dabei werden im wesentlichen alle infrarotaktiven Gase, wie zum Beispiel CO, NO, $SO_2$, $CO_2$, $NH_3$ oder $CH_4$ gemessen.

Ausgehend von diesem Stand der Technik ist es Aufgabe der Erfindung, einen verbesserten Zweistrahl-Gasanalysator bereitzustellen, der eine hohe Langzeitstabilität von Nullpunkt und Empfindlichkeit aufweist, sowie ein verbessertes Verfahren zur Bestimmung von Gaskomponenten in einem Messgas mit dem Zweistrahl-Verfahren bereitzustellen.

Diese Aufgabe wird gelöst durch eine Vorrichtung mit den Merkmalen des Anspruchs 1 bzw. durch ein Verfahren mit den Merkmalen des Anspruchs 7.

Vorteilhafte Ausführungen der erfindungsgemäßen Vorrichtung bzw. des erfindungsgemäßen Verfahrens sind Gegenstand der abhängigen Ansprüche.

Der erfindungsgemäße Zweistrahl-Gasanalysator weist eine Messstrahlungsquelle, eine das zu messende Gas aufnehmbare Messküvette, eine ein Referenzgas enthaltene Referenzküvette, einen Detektor zur Detektion der durch die Küvetten hindurchgetretenen Messstrahlung sowie einen Modulator zum periodischen Umschalten der Messstrahlung von einer Messseite zur Referenzseite und umgekehrt auf, wobei der Modulator so ausgebildet ist, dass innerhalb einer Periode wenigstens zwei Dunkelphasen erzeugt werden.

Die Detektorsignale werden in einer Auswerteeinheit ausgewertet und ein entsprechender Messwert zur Verfügung gestellt. Durch die Dunkelphasen, die mit einer anderen Frequenz auftreten als die Mess- und die Referenzphasen, wird dem Messsignal eine Oberwelle mit anderer Frequenz aufmoduliert. Das modulierte Empfängersignal wird in der Auswerteeinheit ausgewertet, indem eine mit dem Modulator synchronisierte Fouriertransformation erfolgt, woraus sich Sinus- und Cosinusanteil der Grundwelle ergeben, sowie Sinus- und Cosinusanteil der Oberwelle. Mit diesen vier Signalen erfolgt eine Normierung und letztlich der Erhalt des eigentlichen Messwertes. Dadurch kann eine hohe Langzeitstabilität erreicht werden trotz Änderung der Strahlleistung oder Verschmutzungen der Messküvette. Dadurch kann der erfindungsgemäße Gasanalysator ohne Kalibrierküvetten oder Prüfgase lediglich durch eine Einpunktkalibrierung, zum Beispiel mit Umgebungsluft, über Monate hinweg stabil betrieben werden.

Dazu trägt auch bei, dass hochselektive Detektoren einsetzbar sind (z. B. Zweischichtempfänger), die eine minimierte Querempfindlichkeit gegenüber Fremdgasen aufweisen. Bei dem erfindungsgemäßen Gasanalysator bleibt die Empfindlichkeit über lange Zeit erhalten, denn die Verschmutzung wirkt sich nur auf den Nullpunkt aus.

In einer vorteilhaften Weiterbildung der Erfindung sind die Dunkelphasen jeweils vorgesehen bei einem Wechsel von der Messseite zur Referenzseite und umgekehrt. Dadurch wird dem Messsignal eine Oberwelle mit doppelter Frequenz als die Grundwelle aufmoduliert, da die Dunkelphasen mit der doppelten Häufigkeit auftreten als die Messphase bzw. die Referenzphase. Eine doppelte Frequenz der Oberwelle vereinfacht die Auswertung.

Vorteilhafterweise sind die Dauer der Dunkelphasen relativ kurz, beispielsweise etwa 1/12-tel der Periode des Modulators. Die Dunkelphasen werden lediglich dazu benötigt, eine Oberwelle aufzumodulieren, die selbst aber klein bleiben kann, um die Grundwelle, also das eigentliche Messsignal relativ groß zu erhalten.

In einer Weiterbildung der Erfindung ist der Detektor als opto-pneumatischer Zweischichtempfänger ausgebildet, wie er grundsätzlich beispielsweise aus der Zeitschrift "Chemie-Ingenieur-Technik", Heft 16 (1967), Seite 937-945, bekannt ist. Prinzipiell wäre es auch möglich, einen opto-pneumatischen Detektor nach dem Einschichtsystem einzusetzen. Beim Detektor nach dem Zweischichtsystem kann aber bei geeigneter Dimensionierung der Einfluss von Störgasen direkt kompensiert oder vermindert werden.

In vorteilhaft einfacher Weise ist der Modulator als Blendenrad ausgebildet, wobei in konstruktiv günstiger Weise der Wechsel von Messseite zur Referenzseite dadurch realisiert wird, dass das Blendenrad auf zwei Teilkreisen unterschiedlichen Durchmessers Durchbrechungen aufweist, die aus Symmetriegründen diametral angeordnet sind, und die sich jeweils über weniger als 180° erstrecken, so dass zwischen den Durchbrechungen jeweils eine Dunkelphase eintritt, in der das Blendenrad die Messstrahlung blockiert.

Die Erfindung wird im Folgenden anhand von Ausführungsbeispielen unter Bezugnahme auf die Zeichnung im Einzelnen erläutert. In der Zeichnung zeigen:

Fig.1    eine Ausführungsform des erfindungsgemäßen ZweistrahlGasanalysators;

Fig. 2    eine Vorderansicht eines Blendenrades des erfindungsgemäßen Gasanalysators (Schnitt entlang Linie II-II aus Fig. 1);

Fig. 3    eine weitere Ausführungsform;

Fig. 4    ein typisches Messsignal.

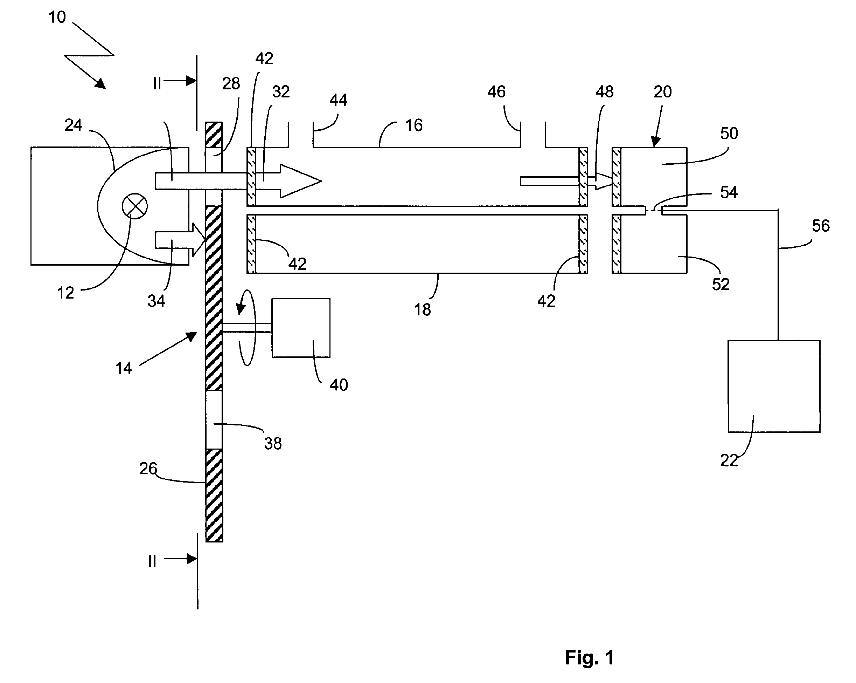

Ein in Figur 1 dargestellter erfindungsgemäßer Zweistrahl-Gasanalysator 10 weist eine Infrarotstrahlungsquelle 12, einen Modulator 14, eine Meßküvette 16, eine Referenzküvette 18, einen Detektor 20 und eine Auswerteeinheit 22 auf.

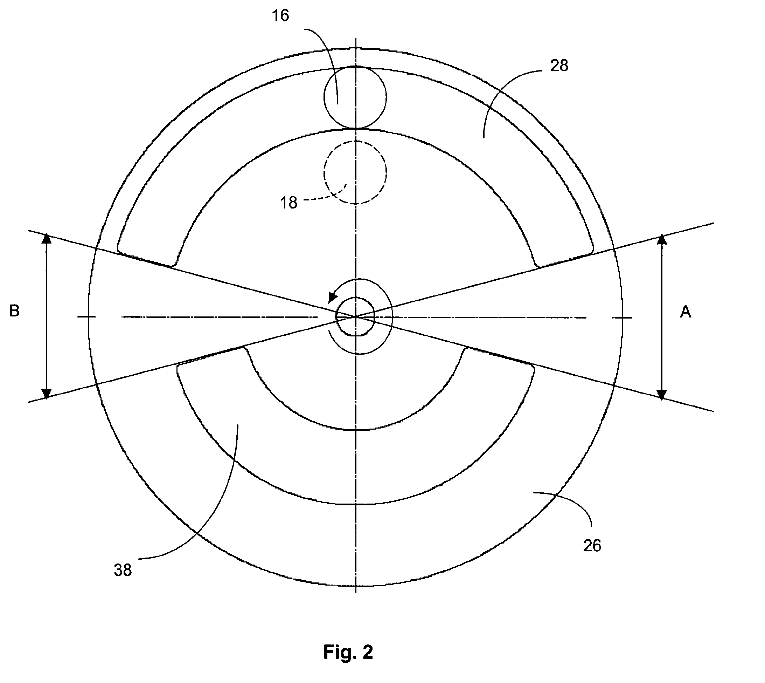

Die von der Strahlungsquelle 12 erzeugte Infrarotstrahlung wird über eine geeignete Strahlformungsoptik 24 in Richtung auf die Messküvette 16 und die Referenzküvette 18 gelenkt. Zwischen der Strahlungsquelle 12 und den Küvetten 16, 18 befindet sich der Modulator 14, der als Blendenrad 26 ausgebildet ist, das in Figur 2 in seiner Vorderansicht dargestellt ist. Das Blendenrad 26 weist eine Messstrahldurchbrechung 28 auf, die auf einem Teilkreis angeordnet ist und sich über weniger als 180° erstreckt. Die Anordnung der Messstrahldruchbrechung 28 auf dem Blendenrad 26 und die Anordnung des Blendenrades 26 zwischen Strahlungsquelle 12 und Messküvette 16 ist so gewählt, dass wenn das Blendenrad 26 sich in der in Figur 1 und 2 dargestellten Stellung befindet, Messstrahlung ausschließlich die Messstrahldurchbrechung 28 durchsetzen kann und in die Messküvette 16 eintreten kann, was durch Pfeil 32 angedeutet ist. In dem Winkelbereich des Blendenrades, in dem die Messstrahldurchbrechung 28 angeordnet ist, wird ein anderer Teil der von der Strahlungsquelle 12 erzeugten Strahlung blockiert, was durch den Pfeil 34 angedeutet ist, so dass wenn die Strahlung die Messseite durchstrahlt kein Licht in die Referenzküvette 18 eintreten kann.

Diametral der Messstrahldurchbrechung 28 gegenüberliegend ist auf einem kleineren Teilkreis 36 eine Referenzstrahldurchbrechung 38 in analoger Weise angeordnet, die sich über einen gleich großen Winkelbereich erstreckt wie die Messstrahldurchbrechung 28. Bei entsprechender Stellung des Blendenrades 26, wenn die Strahlung die Referenzseite durchsetzt, ist dann die Messseite blockiert und die Strahlung der Strahlungsquelle 12 kann nur in die Referenzküvette 18 eintreten.

Da die Durchbrechungen 28 und 38 sich jeweils über weniger als 180° erstrecken, sind zwei diametral gegenüberliegende Winkelbereiche A und B gegeben, in denen sowohl die Messseite als auch die Referenzseite abgedeckt sind, so dass auf keiner Seite Strahlung in die Küvetten gelangen kann. Dadurch sind Dunkelphasen definiert, in denen keine Strahlung bis zum Detektor 20 gelangen kann.

Der Teil der Strahlung, der von dem Blendenrad durchgelassen wird, tritt je nach Stellung des Blendenrades, das über einen Motor 40 kontinuierlich angetrieben wird, entweder in die Messküvette 16 oder die Referenzküvette 18 ein. Die Küvetten sind endseitig durch infrarotdurchlässige Fenster 42 abgeschlossen. In der Referenzküvette 18 befindet sich ein infrarotinaktives Referenzgas und in der Messküvette 16 ist das zu messende Gas mit der infrarotaktiven Komponente enthalten, wobei das Gas über eine Zufuhrleitung 44 und eine Abfuhrleitung 46 durch die Messküvette 16 geleitet werden kann.

Die die Küvetten durchlaufende Strahlung tritt in einen an sich bekannten opto-pneumatischen Detektor 20 ein, wie durch Pfeil 48 angedeutet ist. In dem Ausführungsbeispiel nach Figur 1 ist der opto-pneumatische Detektor als Einschichtsystem dargestellt. Der Detektor 20 enthält ein infrarotaktives Gas, so dass je nach empfangener Strahlung in der jeweiligen Kammer 50 bzw. 52 sich ein Druck und eine Temperatur einstellen wird, die der empfangenen Infrarotstrahlung entspricht. Da auf der Messseite aufgrund des zu messenden Gases eine höhere Absorption der Infrarotstrahlung stattfindet als auf der Referenzseite, wird sich zwischen den Kammern 50 und 52 eine Druck- und Temperaturdifferenz aufbauen, die mittels einer zwischen den Kammern angeordneten Messvorrichtung 54 gemessen wird und an die Auswerteeinheit 22 über eine Leitung gegeben wird.

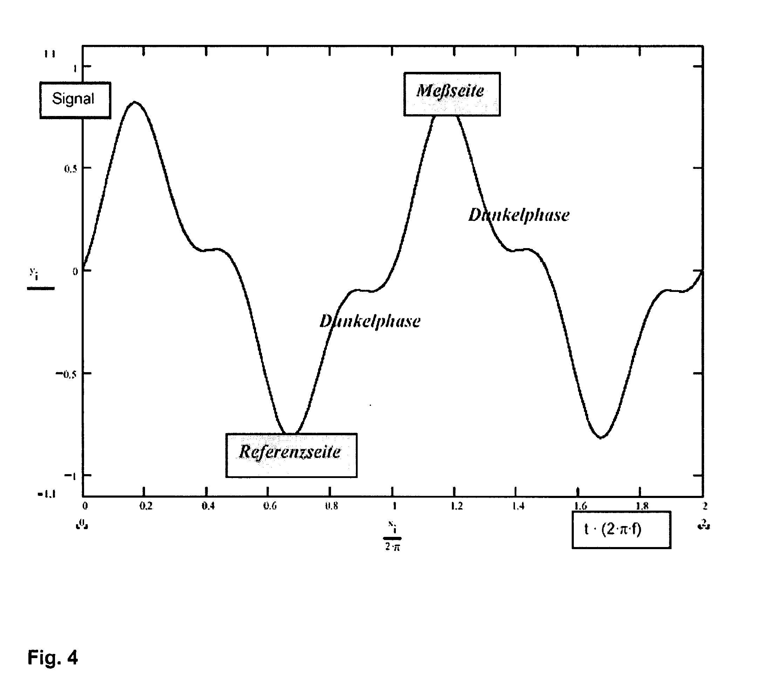

Ein typisches Messsignal, wie es an der Leitung 56 ansteht, ist in Figur 4 dargestellt. Als Grundwelle ist ein sinusförmiger Verlauf der Messkurve zu erkennen, der dadurch entsteht, dass das Blendenrad aufgrund der Durchbrechungen 28 und 38 jeweils nahezu eine halbe Periode die Messseite und für nahezu eine halbe Periode die Referenzseite freigibt, so dass für nahezu eine halbe Periode die Strahlung die Messküvette durchsetzt und in die Kammer 50 des Detektors 20 eintritt und für nahezu die andere halbe Periode die Messstrahlung die Referenzküvette durchsetzt und in die Kammer 52 des Detektors 20 eintritt. Dadurch entstehen periodische Druck -und Temperaturschwankungen, die durch den sinusförmigen Verlauf wiedergegeben werden. Die typische Frequenz beträgt 1 bis 10 Hz und wird von dem Motor 40 des Blendenrades 26 vorgegeben.

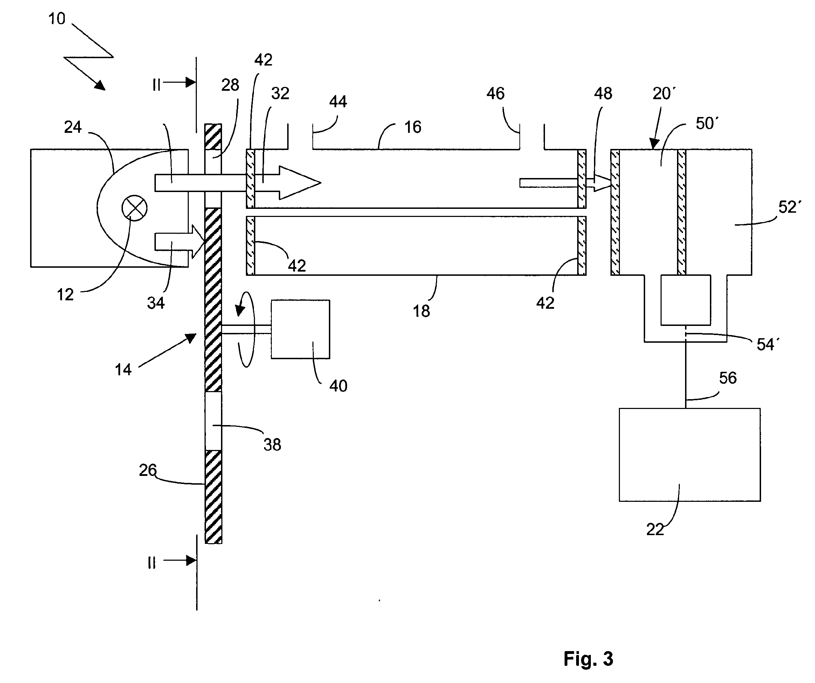

In Fig. 3 ist eine Ausführungsform des erfindungsgemäßen Gasanlysators dargestellt mit einem opto-pneumatischen Detektor 20' nach dem Zweischichtsystem. Auch hier enthalten die beiden Kammern 50'und 52' des Detektors 20', die jetzt hintereinander angeordnet sind, ein infrarotaktives Gas, so dass sich wiederum zwischen den Kammern 50'und 52' eine messbare Druckund Temperaturdifferenz aufbaut, die abhängig ist von der Intensität der einfallenden Strahlung.

Wie bereits erläutert, sind durch das Blendenrad 26 zwischen dem Umschalten von der Messseite auf die Referenzseite und umgekehrt die genannten Dunkelphasen vorgesehen, in denen kein Licht auf den Detektor 20 trifft und in denen im Detektor 20 das Gas relaxieren kann.

Diese Dunkelphasen treten mit doppelter Frequenz auf als die der sinusförmigen Grundwelle, was sich im Messsignal dadurch äußert, dass der sinusförmigen Grundwelle eine Oberwelle mit doppelter Frequenz aufmoduliert ist, was in Figur 4 dargestellt ist.

Der sinusförmige Verlauf des Messsignals ergibt sich dadurch, dass der Detektor eine gewisse Trägheit besitzt und der Strahlmodulation des Modulators nicht unmittelbar folgen kann. Aus diesem Grunde sind auch die Dunkelphasen gegenüber dem Nullpunkt leicht verschoben, denn die Lage des Signals während der Dunkelphase hängt davon ab, ob der Detektor zuvor Strahlung von der Messseite oder Strahlung von der Referenzseite aufgenommen hat.

Das in Figur 4 dargestellte Signal wird in der Auswerteeinheit 22 einer Fourieranalyse unterzogen, womit Amplitude und Phase von Grund- und Oberwelle bestimmt sind:

$$\{1.1\} \quad A_f \ \cos(\phi_f)$$

$$\{2.1\} \quad A_{2f} \ \cos(\phi_{2f})$$

$$\{1.2\} \quad A_f \ \sin(\phi_f)$$

$$\{2.2\} \quad A_{2f} \ \sin(\phi_{2f}),$$

wobei A die Amplitude, f die Frequenz der Grundwelle und 2f die Frequenz der Oberwelle bedeuten.

Die Wurzel aus der Summe der Quadrate der Signale (2.1) und (2.2) ergibt die Normierungsgröße $A_{2f}$ (Amplitude der Oberwelle). Nach Division der Signale (1.1) und (1.2) durch die Normierungsgröße folgen die normierten Messgrößen:

$$\{3.1\} \quad x = A_f \ / \ A_{2f} \ \cos(\phi_f)$$

$$\{3.2\} \quad y = A_f \ / \ A_{2f} \ \sin(\phi_f).$$

Die Messgrößen werden anschließend durch eine Reihe von Koordinatentransformationen so umgewandelt, dass sich ein einfaches Handling ergibt und die eigentlich zu messende Messgröße, nämlich die Konzentration der zu messenden Gaskomponente in dem Messgas erhalten werden kann.

**Patentansprüche**

1. Zweistrahl-Gasanalysator mit einer Messstrahlungsquelle (12), mit einer ein zu messendes Gas aufnehmbaren Messküvette (16), mit einer ein Referenzgas enthaltenden Referenzküvette (18), mit einem Detektor (20) zur Detektion der von der Messstrahlungsquelle (12) abgegebenen Messstrahlung (32, 34), mit einem Modulator (14) zum periodischen Umschalten der Messstrahlung von der Messseite auf die Referenzseite und umgekehrt und mit einer Auswerteeinheit (22), **dadurch gekennzeichnet, dass** der Modulator (14, 26) ausgebildet ist um innerhalb einer Periode wenigstens zwei Dunkelphasen zu erzeugen.

2. Zweistrahl-Gasanalysator nach Anspruch 1, **dadurch gekennzeichnet, dass** die Dunkelphasen beim Wechsel von der Messseite auf die Referenzseite bzw. umgekehrt liegen.

3. Zweistrahl-Gasanalysator nach einem der vorhergehenden Ansprüche, **dadurch gekennzeichnet, dass** die Dauer der Dunkelphasen etwa 1/12-tel der Periode beträgt.

4. Zweistrahl-Gasanalysator nach einem der vorhergehenden Ansprüche, **dadurch gekennzeichnet, dass** der Modulator (14) als Blendenrad (26) ausgebildet ist.

5. Zweistrahl-Gasanalysator nach Anspruch 4, **dadurch gekennzeichnet, dass** das Blendenrad (26) auf zwei Teilkreisen unterschiedlichen Durchmessers Durchbrechungen (28 und 38) aufweist, die sich jeweils über weniger als 180° erstrecken und diametral angeordnet sind.

**6.** Zweistrahl-Gasanalysator nach einem der vorhergehenden Ansprüche, **dadurch gekennzeichnet, dass** der Detektor (20) als opto-pneumatischer Zweischichtempfänger ausgebildet ist.

**7.** Verfahren zur Bestimmung von Gaskomponenten in einem Messgas mit dem Zweistrahl-Verfahren mit folgenden Schritten:

- Bereitstellen einer Messstrahlung,
- periodisch abwechselndes Führen der Messstrahlung zur Messseite und zur Referenzseite, wobei innerhalb einer Periode einmal das Messgas und einmal das Referenzgas durchstrahlt wird.
- Detektieren der durch das Messgas bzw. das Referenzgas durchgelassenen Messstrahlung,

**dadurch gekennzeichnet, dass** wenigstens zwei Dunkelphasen vorgesehen sind, während denen die Messstrahlung blockiert wird.

**8.** Verfahren nach Anspruch 8, **dadurch gekennzeichnet, dass** zwischen dem Umschalten von der Messseite auf die Referenzseite und umgekehrt jeweils eine Dunkelphase vorgesehen ist.

Fig. 1

Fig. 2

Fig. 3

Fig. 4

**Europäisches Patentamt**

# EUROPÄISCHER RECHERCHENBERICHT

Nummer der Anmeldung

EP 04 02 2907

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | DE 20 03 247 A1 (ERNST LEITZ GMBH) 29. Juli 1971 (1971-07-29) * Abbildung 1 * * Seite 1, Zeile 1 - Zeile 6 * * Seite 2, Zeile 28 - Seite 3, Zeile 8 * ----- | 1-8 | G01N21/35 G01N21/37 |
| A | US 3 162 761 A (LUFT KARL) 22. Dezember 1964 (1964-12-22) * Abbildungen 1-3 * * Spalte 2, Zeile 49 - Zeile 60 * * Spalte 4, Zeile 34 - Zeile 64 * ----- | 1-8 | |
| A | US 3 937 962 A (FAULHABER ET AL) 10. Februar 1976 (1976-02-10) * Zusammenfassung; Abbildung 1 * * Spalte 3, Zeile 11 - Zeile 15 * ----- | 1-8 | |
| D,A | EP 0 780 681 A (SIEMENS AKTIENGESELLSCHAFT) 25. Juni 1997 (1997-06-25) * Zusammenfassung; Abbildung 1 * * Spalte 2, Zeile 40 - Spalte 3, Zeile 23 * ----- | 1-8 | |

| RECHERCHIERTE SACHGEBIETE (Int.Cl.7) |
|---|
| G01N G01J |

Der vorliegende Recherchenbericht wurde für alle Patentansprüche erstellt

| Recherchenort | Abschlußdatum der Recherche | Prüfer |
|---|---|---|
| Den Haag | 28. Januar 2005 | Bockstahl, F |

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 04 02 2907

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-01-2005

| Im Recherchenbericht angeführtes Patentdokument | | Datum der Veröffentlichung | Mitglied(er) der Patentfamilie | | Datum der Veröffentlichung |
|---|---|---|---|---|---|
| DE 2003247 | A1 | 29-07-1971 | KEINE | | |
| US 3162761 | A | 22-12-1964 | CH | 374495 A | 15-01-1964 |
| | | | DE | 1302592 B | 09-11-1972 |
| | | | FR | 1291241 A | 20-04-1962 |
| | | | GB | 979850 A | 06-01-1965 |
| US 3937962 | A | 10-02-1976 | DE | 2359637 A1 | 05-06-1975 |
| | | | JP | 50087692 A | 14-07-1975 |
| EP 0780681 | A | 25-06-1997 | DE | 19547787 C1 | 17-04-1997 |
| | | | EP | 0780681 A2 | 25-06-1997 |
| | | | US | 5764354 A | 09-06-1998 |

Für nähere Einzelheiten zu diesem Anhang : siehe Amtsblatt des Europäischen Patentamts, Nr.12/82